# EUROPEAN PATENT SPECIFICATION

(11) **EP 3 829 428 B1**
(45) Date of publication and mention of the grant of the patent: **02.12.2026**
(21) Application number: 19773536.8
(22) Date of filing: 25.07.2019
(51) Int. Cl.: A61B 5/01, A61B 18/14, A61B 5/287, A61B 5/318, H05K 1/11, H05K 1/189, A61B 5/00, A61B 18/00, A61B 17/00

(54) **FLEXIBLE-CIRCUIT TIP FOR A SPLIT-TIP CATHETER**
FLEXIBLE KREISLAUFSPITZE FÜR EINEN KATHETER MIT GETEILTER SPITZE
POINTE À CIRCUIT SOUPLE POUR CATHÉTER À POINTE FENDUE

(30) Priority: 30.07.2018 US 201862711708 P; 02.07.2019 US 201916502020
(43) Date of publication of application: 09.06.2021
(73) Proprietor: Biosense Webster (Israel) Ltd., Yokneam, 2066717 (IL)
(72) Inventor: RAO, Anand, Irvine, CA 92618 (US); DATTA, Keshava, Irvine, CA 92618 (US); BEECKLER, Christopher T., Irwindale, CA 91706 (US); PENDEKANTI, Rajesh, Irvine, CA 92618 (US)
(74) Representative: Carpmaels & Ransford LLP
(86) International application number: PCT/IB2019/056381
(87) International publication number: WO 2020/026096

(56) References cited:
- EP-A1- 3 300 680
- EP-A1- 3 315 087
- EP-A1- 3 572 024
- WO-A2-2011/143468

## Description

### FIELD

The subject matter disclosed relates to electrophysiology catheters and more particularly to a flexible-circuit tip for a split-tip catheter for use in electrocardiology ablation and mapping procedures.

### BACKGROUND

Cardiac arrhythmias, such as atrial fibrillation, occur when regions of cardiac tissue abnormally conduct electric signals to adjacent tissue, thereby disrupting the normal cardiac cycle and causing asynchronous rhythm.

Procedures for treating arrhythmia include surgically disrupting the origin of the signals causing the arrhythmia, as well as disrupting the conducting pathway for such signals. By selectively ablating cardiac tissue by application of energy via a catheter, it is sometimes possible to block or modify the propagation of unwanted electrical signals from one portion of the heart to another. The ablation process destroys the unwanted electrical pathways by formation of non-conducting lesions.

For further background, EP 3315087A describes a catheter that includes an insertion tube, a flexible substrate and one or more electrical devices. The insertion tube is configured for insertion into a patient body. The flexible substrate is configured to wrap around a distal end of the insertion tube and includes electrical interconnections. The electrical devices are coupled to the flexible substrate and are connected to the electrical interconnections.

### SUMMARY OF THE INVENTION

The invention provides a flexible circuit according to claim 1 and a method of assembling a catheter according to claim 14. Further embodiments are defined in the dependent claims. The disclosed methods of using a catheter (i.e., device) for treatment of patients are described with reference to the device (and method of assembly of the device) of the present invention. Whilst the method of treatment is not explicitly recited in the wording of the claims, the claimed device is capable of carrying out the corresponding operations.

### SUMMARY OF THE DISCLOSURE

Ablation, particularly of cardiac tissue, depends upon accurate delivery of ablative energy while avoiding negative side effects caused by providing ablative energy to blood such as thrombus formation. A catheter having a tip divided into three segments directed to these purposes is disclosed. The tip may be a fabricated as a planar flexible circuit, e.g., via lithography, as having a first planar segment and a second planar segment. The second planar segment may comprise a plurality of irrigation ports disposed therethrough. The second planar segment may also comprise a first layer including a substrate, a second layer including at least a first temperature sensor, a second temperature sensor, and a conductor element, and a third layer including an insulator (e.g., a polyamide, polyimide, or polyurethane material). Additionally, the second planar segment may include a first sector and a second sector, the first sector having the first temperature sensor and the second temperature sensor, and the second sector having a third temperature sensor and a fourth temperature sensor. The second planar segment may also include a third sector, the third sector having a fifth temperature sensor and a sixth temperature sensor. In any of these embodiments, the conductor element may include a trace connected to an ablation electrode. Additionally, the first sector, the second sector, and the third sector may each include a respective solder pad having a first contact operatively coupled to a respective thermocouple, a second contact operatively coupled to another respective thermocouple, and a third contact operatively coupled to a respective electrode.

Also in any of these embodiments, the first planar segment may include a first-segment substrate and a first-segment insulator. Further, the first planar segment may include a first-segment temperature sensor. It may also include a first-segment electrode. A first space may be provided between the second layer of the first sector and the second layer of the second sector, and a second space may be provided between the second layer of the second sector and the second layer of the third sector. A first insulation material may be disposed within the first space, and alternatively or additionally within the second space. The first insulation material may be a suitable insulation material such as, for example, bio-compatible ceramics or a high-temperature epoxy.

The tip, in any of the foregoing embodiments, may be included on a distal end of a catheter. The catheter may also include an elongate body having at least two lumens disposed longitudinally therethrough. A core may be attached to the distal end of the catheter, at least a portion of which may be disposed within the second segment of the tip. The core may comprise an insulative material, such as polyurethane. Further, the core may include a lumen oriented transverse to a longitudinal axis of the core. A second insulation material may be disposed between the second segment and the core. The core may be in communication with a first one of the at least two lumens of the catheter body such that fluid may flow through one of the lumens and through the core. A plurality of wires may be disposed within at least a second one of the at least two lumens and this plurality of wires may be electrically connected to the flexible-circuit tip.

The catheter may be assembled by first receiving the catheter body and the tip in a planar configuration, as fabricated. The tip may have its planar configuration changed to a non-planar configuration (e.g., cylindrical configuration) by bending the tip as such and then connecting it to the distal end of the catheter body. In those embodiments that include a core, the core may be received and then attached to the distal end of the catheter body. The core may then be disposed within the tip in the cylindrical configuration, and then attached to the tip.

The catheter may be used according to the following method and variations. First, the catheter may be inserted into a subject, e.g., a human subject, proximate to the subject's heart. The tip may be maneuvered into contact with the tissue. The catheter may be an aspect of an ablation system that also includes a processor that is in communication with the tip. The first sector may monitor an ECG signal and provide the signal to the processor. The second sector may monitor an ECG signal and provide the signal to the processor. The third sector may monitor an ECG signal and provide the signal to the processor. Each of the three sectors may also measure temperature and provide temperature data to the processor. Ablation energy may be provided to the tip, e.g., as controlled by the processor.

In some variations of the method, the processor determines that the first tip sector contacts tissue. Further, the processor may determine that the second tip sector contacts tissue. Further, the processor may determine that the third tip sector contacts tissue.

The processor may control ablation energy to the first tip sector while receiving ECG signals from the second tip sector at the processor. Such may also be performed while the processor receives ECG signals from the third tip sector at the processor. The processor may control ablation energy to the second tip sector while receiving ECG signals from the third tip sector. Such may also be performed while the processor receives ECG signals from the first tip sector. The processor may control ablation energy to the third tip sector while receiving ECG signals from the first tip sector at the processor. Such may also be performed while the processor receives ECG signals from the second tip sector. Additionally or alternatively, the processor may control ablation energy simultaneously to at least two of the first, second, and third tip sectors.

In any of these variations, a portion of tissue in contact with the first tip sector may be ablated. Then, without moving the tip a portion, tissue in contact with the second tip sector may be ablated.

As used herein, the terms "insulator," "insulation material," "insulative material," and the like, each connote materials and structures comprising at least one material that has properties, generally accepted by those of skill in the art, to resist transfer of heat and conveyance of electrical signals. Such materials include, but are not limited to, polyamide, polyimide, polyurethane, polycarbonate, ceramic, liquid crystal polymer, and high-temperature epoxy.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
Figure 1 is a pictorial illustration of a system for evaluating electrical activity in a heart of a living subject and providing treatment thereto using a catheter;
Figure 2 depicts a flexible circuit;
Figure 3 depicts the flexible circuit of Figure 2 formed into a flexible-circuit tip and connected to a distal end of a catheter;
Figure 4 is a representation of Figure 3, with the flexible-circuit tip hidden;
Figure 5 depicts an alternative embodiment of a catheter including a flexible-circuit tip;
Figure 6 depicts another flexible circuit;
Figure 7 depicts the flexible circuit of Figure 6 in a modified configuration;
Figure 8 depicts yet another flexible circuit;
Figure 9 depicts a spring component;
Figure 10 depicts the distal portion of the catheter of Figure 3 in a partially assembled configuration;
Figure 11 depicts the distal portion of the catheter of Figure 3 in a further partially assembled configuration; and
Figure 12 depicts a cross section taken through line A-A of Figure 10.

### MODES OF CARRYING OUT THE INVENTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

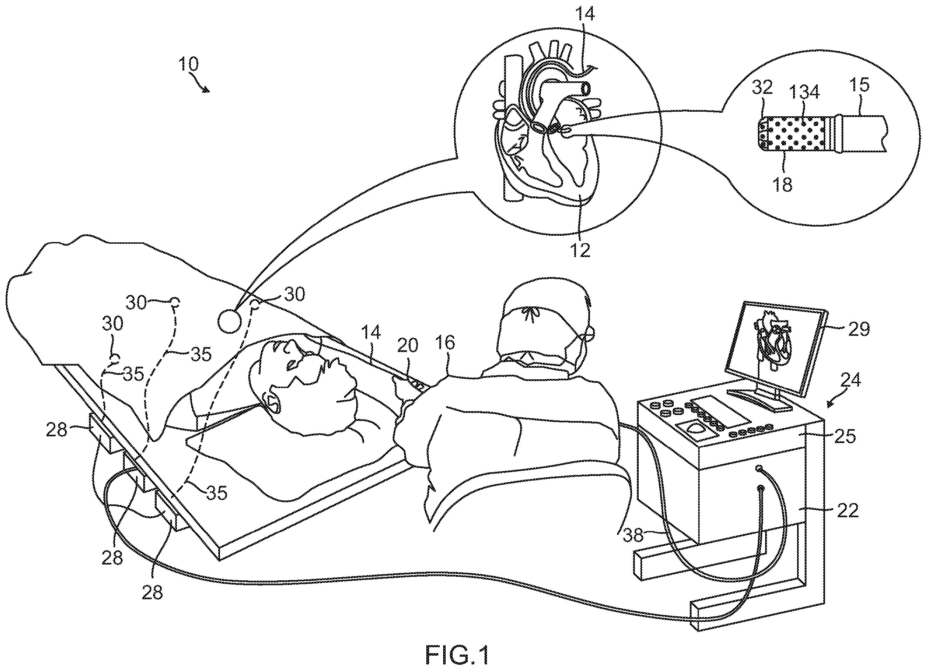

Figure 1 is a pictorial illustration of a system 10 for evaluating electrical activity and performing ablative procedures on a heart 12 of a living subject. The system includes a diagnostic/therapeutic catheter having a catheter body 14 having a distal end 15 and a tip, e.g., tip18 disposed thereon, which may be percutaneously inserted by an operator 16 through the patient's vascular system into a chamber or vascular structure of the heart 12. The operator 16, who is typically a physician, brings the catheter's tip 18 into contact with the heart wall, for example, at an ablation target site. Electrical activation maps may be prepared, according to the methods disclosed in U.S. Patent Nos. 6,226,542, and 6,301,496, and in commonly assigned U.S. Patent No. 6,892,091. One commercial product embodying elements of system 10 is available as the CARTO^{®} 3 System, available from Biosense Webster, Inc., 33 Technology Drive, Irvine, CA 92618.

Areas determined to be abnormal, for example by evaluation of the electrical activation maps, can be ablated by application of thermal energy, e.g., by passage of radiofrequency electrical current through wires in the catheter to one or more electrodes at the tip 18, which apply the radiofrequency energy to target tissue. The energy is absorbed in the tissue, heating it to a point (typically above 50°C) at which point it permanently loses its electrical excitability. This procedure creates non-conducting lesions in the cardiac tissue, which disrupt the abnormal electrical pathway causing the arrhythmia. Such principles can be applied to different heart chambers to diagnose and treat many different types of cardiac arrhythmias.

The catheter typically includes a handle 20, having suitable controls on the handle to enable the operator 16 to steer, position and orient the distal end 15 of the catheter as desired for the ablation.

Ablation energy and electrical signals can be conveyed to and from the heart 12 through one or more electrodes 32 located at or near the tip 18, or comprising tip 18, via cable 38 to the console 24. Pacing signals and other control signals may be conveyed from the console 24 through the cable 38 and the electrodes 32 to the heart 12.

Wire connections 35 link the console 24 with body surface electrodes 30 and other components of a positioning sub-system for measuring location and orientation coordinates of the catheter. The processor 22 or another processor may be an element of the positioning subsystem. The electrodes 32 and the body surface electrodes 30 may be used to measure tissue impedance at the ablation site as taught in U.S. Patent No. 7,536,218, issued to Govari et al.. At least one temperature sensor, typically a thermocouple or thermistor, may be included on or near each of the electrodes 32, as will be detailed below.

The console 24 typically contains one or more ablation power generators 25. The catheter may be adapted to conduct ablative energy to the heart using any known ablation technique, e.g., radiofrequency energy, ultrasound energy, cryogenic energy, and laser-produced light energy. Such methods are disclosed in commonly assigned U.S. Patent Nos. 6,814,733, 6,997,924, and 7,156,816.

The positioning subsystem may also include a magnetic position tracking arrangement that determines the position and orientation of the catheter by generating magnetic fields in a predefined working volume and sensing these fields at the catheter, using coils or traces disposed within the catheter, typically proximate to the tip. A positioning subsystem is described in U.S. Patent No. 7,756,576 and in the above-noted U.S. Patent No. 7,536,218.

Operator 16 may observe and regulate the functions of the catheter via console 24. Console 24 includes a processor, preferably a computer with appropriate signal processing circuits. The processor is coupled to drive a monitor 29. The signal processing circuits typically receive, amplify, filter and digitize signals from the catheter, including signals generated by sensors, e.g., electrodes 32, such as electrical and temperature sensors, and a plurality of location sensing coils or traces located distally in the catheter. The digitized signals are received and used by the console 24 and the positioning system to compute the position and orientation of the catheter, and to analyze the electrical signals received from the catheter.

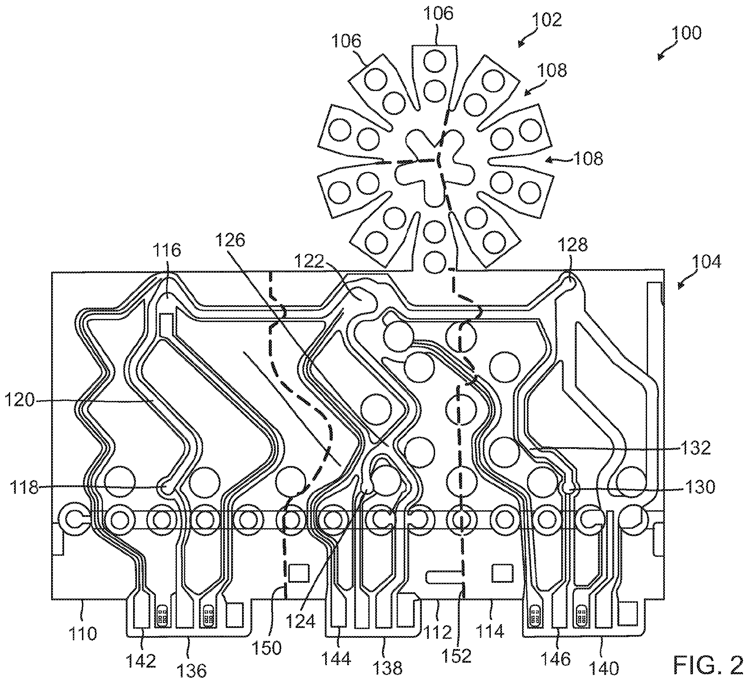

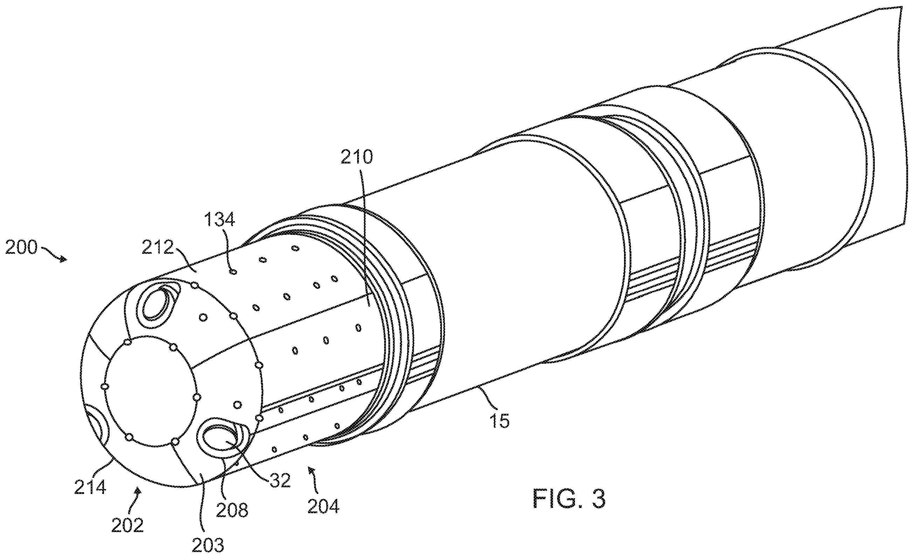

The subject matter disclosed herein concerns improvements to fabrication and functionality of catheter tips known in the art, such as that disclosed in U.S. Patent No. 6,171,275 to Webster. The improved catheter tip may be fabricated via a lithographic process as a planar flexible circuit 100 reflected in Figure 2. The flexible circuit 100 is, as its description suggests, flexible. Accordingly, it may be bent into various non-planar configurations. For example, the configuration may be changed from planar to cylindrical, such that flexible circuit 100 may be changed into a cylindrical flexible-circuit tip 200, reflected in Figure 3. Accordingly, apart from the planar configuration of flexible circuit 100 and the non-planar configuration of flexible-circuit tip 200, it should be understood that features described herein with respect to flexible circuit 100 are also present in flexible-circuit tip
200 and, similarly, features described herein with respect to flexible-circuit tip 200 are also present in flexible circuit 100, even if express disclosure is not made concerning one of these configurations. Further, the surface of flexible circuit 100 visible in Figure 2 becomes the inner surface of flexible-circuit tip 200, and thus the electronic componentry visible in Figure 2 is not visible in Figure 3

Flexible circuit 100 may include various segments depending on the desired structure of the flexible-circuit tip into which it will be formed. As seen in Figure 2, flexible circuit 100 has two segments, i.e., a first segment 102 and a second segment 104. First segment 102 may have a circular shape and second segment 104 may have a rectangular shape. So comprised, flexible circuit 100 may be formed into the cylindrical flexible-circuit tip 200 reflected in Figure 3, with first segment 102 becoming the distal-most portion (base of the cylinder) 202 of tip 200, i.e., a, and with segment 104 becoming a lateral surface (wall of the cylinder) 204 of tip 200.

First segment 102 may be provided as having a geared or floral pattern comprising teeth or petals 106. Spaces 108 between teeth 106 may accommodate a transition zone 203 between base 202 and wall 204. Holes may further be provided through first segment 102, such that holes 208 would be disposed within transition zone 203. Holes 208 may accommodate various electronic components of the catheter, e.g., electrodes 32. Additional electronic components may be incorporated into first segment 102, such as a temperature sensor (e.g., a thermocouple), described below.

Second segment 104 may include at least two, e.g., three, sections or sectors, such as first sector 110, second sector 112, and third sector 114. Dotted lines are provided on second segment 104 demarcating boundaries between these sectors. Dotted lines are also provided on first segment 102 demarcating portions that align with sectors 110, 112, and 114 in flexible-circuit tip 200.

First sector 110 may include two temperature sensors (e.g., thermocouples) 116 and 118, and a conductor element 120. Second sector 112 may include two temperature sensors 122 and 124, and a conductor element 126. Third sector 112 may include two temperature sensors 128 and 130, and a conductor element 132. Conductor elements 120, 126, and 132 may each comprise at least a trace. Alternatively or additionally, conductor elements 120, 126, and 132 may additionally comprise or be connected to an electrode, which may be, for example, conductive portions of first, second, and third sectors 110, 112, and 114, particularly the outer layer of second segment 104 that becomes the outer surface of tip 200. In such embodiments, vias should be provided between conductor elements 120, 126, and 132 and the outer layer. Alternatively or additionally conductor elements 120, 126, and 132 may be connected to an electrode (e.g., electrodes 32). The electrodes (whether the outer surface of tip 200 or electrodes 32) may function as, e.g., ablation electrodes, mapping electrodes, or a combination thereof depending on whether they receive signals from generators 25 or detect electric signals from tissue that they may provide to processor 22.

Flexible circuit 100 may further comprise various layers, e.g., formed via a lithographic process. At least one layer may be an electrically conductive material, e.g., gold, platinum, or palladium, or a combination thereof. For example, the layer that forms the outer surface seen in Figure 3 on flexible-circuit tip 200, may be formed of electrically conductive material. Additionally, another layer that includes at least some of the electronic componentry of Figure 2 (e.g., temperature sensors 116 and 118) may also be formed of electrically conductive material. Another layer may comprise a substrate, e.g., a thin film of a non-conductive or insulation material, onto which conductive material may be deposited. An additional layer may also comprise an insulator. The substrate layer and the insulating layer are similar, and may be provided as a single layer, however, improved insulation properties may be achieved by providing a layer that has the sole purpose of insulating heat and electronic signals from one portion of tip 200 to other portions of tip 200, as described below.

Ports 134 may be provided through flexible circuit 100. These ports may be used to provide irrigation out of tip 200. Solder pads 136, 138 and 140 may also be provided on second segment 104, i.e., solder pad 136 on first sector 110, solder pad 138 on second sector 112, and solder pad 140 on third sector 114, each having various contacts 142, 144, and 146 that are in conductive communication (operatively coupled) with the electronic componentry disposed on the corresponding sector. That is, for example, solder pad 136 includes various contacts that are operatively coupled to thermocouples 116 and 118, and conductor element 120. In this manner, the electronic componentry on one of the three sectors of second segment 104 may be controlled (e.g., for providing ablation or detecting electronic signals from tissue) and monitored separately (e.g., detecting separate temperatures for the separate temperature sensors disposed on each section of segment 104) from the electronic componentry on the other two sectors of second segment 104. Further, temperatures may be precisely monitored about tip 200 because each of the three sectors includes two distinct temperature sensors, for a total of six temperature sensors on tip 200.

In further embodiments, spacing may be provided between first sector 110 and second sector 112 as well as between second sector 112 and third sector 114. The spacing may be provided through each layer, i.e., through the entire thickness of flexible circuit 100. However, this spacing may be provided through only the layers comprising conductive materials and need not be provided in the substrate and insulating layers comprising non-conductive materials. This spacing may, for example, be provided along the contours identified by the dotted lines 150 and 152 in Figure 2. The spacing segregates the various sectors 110, 112, and 114 from each other, for example, helping prevent distribution of heat from one sector to the other. Accordingly, insulative materials may be disposed within the spacing.

Flexible circuit 100 may be formed into flexible-circuit tip 200 and connected to distal end 15 of catheter body 14. Catheter body may have disposed longitudinally therethrough at least two lumens. For example, one of the two lumens may be used to conduct irrigation fluid through catheter body and into tip 200. The other one of the two lumens may contain lead wires for conveying signals, e.g., electrical signals, to and from the electronic componentry of tip 200. Additional lumens may be provided to, e.g., enable steering functionality, such as by including puller wires, or for a guide wire, as is known in the art.

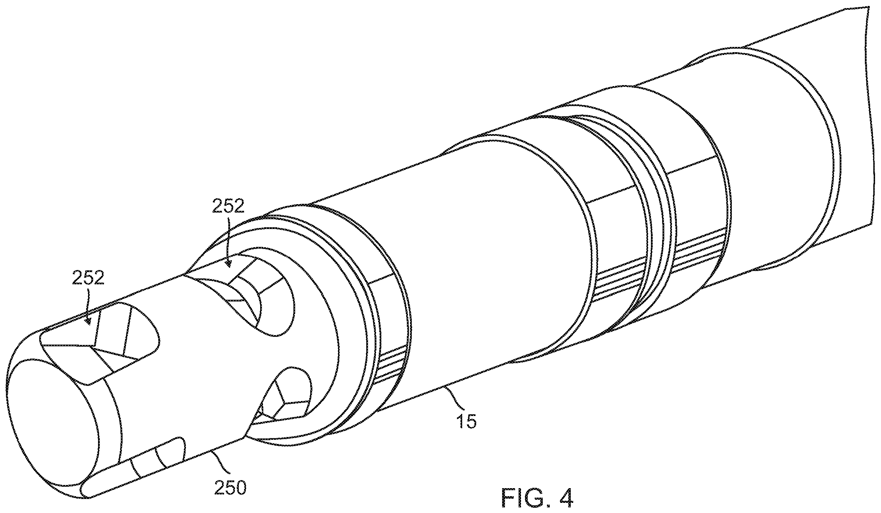

Referring to Figure 4, which is a representation of Figure 3 with tip 200 hidden, a core 250 (Fig. 4) may be attached to distal end 15 of catheter body 14. Core 250 may be disposed within tip 200 and attached thereto such that tip 200 may be connected to distal end 200 via or with the assistance of core 250. Core 250 may include various ports 252 therethrough such that when core 250 is connected to catheter body 14, ports 252 are in fluid communication with the irrigation lumen of catheter body 14. Core 250 may provide the following advantages. First, it may prevent irrigation fluid from entering an interior of tip 200 in a longitudinal direction, which could bias irrigation flow out of irrigation holes. Core 250 instead equalizes the flow distribution by diverting the flow into various streams symmetric to each other and transverse to the core. Second, core 250 may comprise an insulative material, e.g., polycarbonate, which may further assist in preventing heat distribution between the three sectors 210, 212, and 214 of wall 204 (corresponding to the three sectors 110, 112, and 114 of second segment 104 of flexible circuit 100). In this regard, further insulation material may be disposed within a space between core 250 and tip 200, e.g., a high temperature epoxy, polyurethane, polyamide, or polyimide. Third, a portion of core 250 may function as a mandrel about which flexible circuit 100 may be formed into flexible-circuit tip 200.

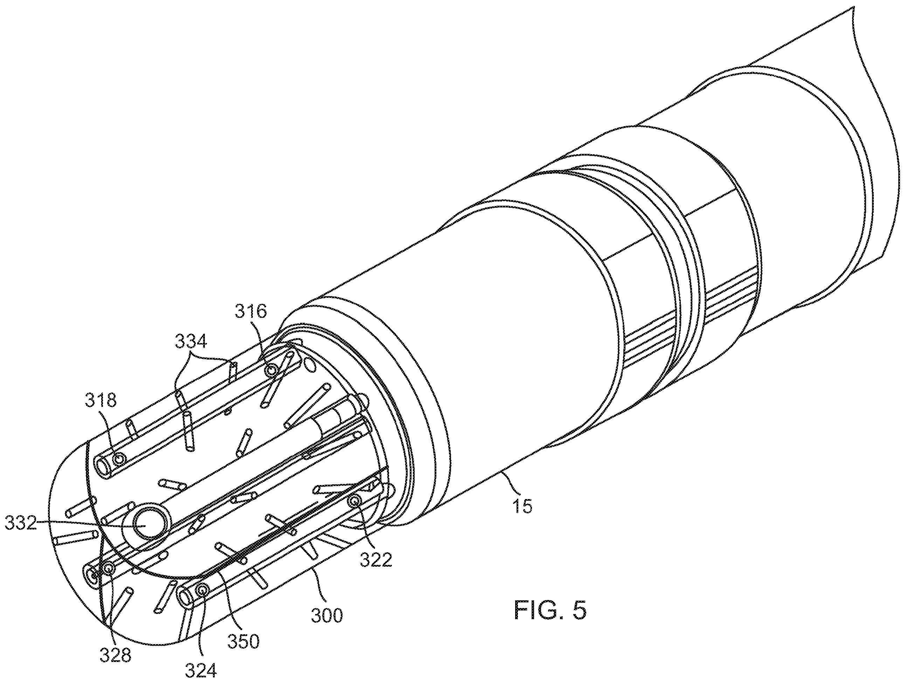

This third advantage may be perceived in Figure 5, which shows an alternative embodiment in which flexible-circuit tip 300 is transparent (except that reference lines are provided to distinguish sectors 310, 312, and 314 from each other). Core 350 is shown therein, also transparent, but with various irrigation ports indicated therethrough. Core 350 thus takes up the entire or most of the entire interior space defined by flexible-circuit tip 300. Thus, in this embodiment, flexible circuit 100 may be molded or conformed to the exterior shape of core 350. Further, it may be bonded thereto, which may facilitate assembly to catheter body 14. Various tubes, e.g., 360, 362, and 364 may also be provided within core 350 to enable couplings to temperature sensors 316, 318, 322, 324, 328, and 330. Additional tubes, e.g., 366 may also be provided within the core to enable couplings to other electronic componentry, e.g., electrode 332. In various embodiments, core 250 and 350 need not include any lumens therethrough such that it might not provide the flow diversion functionality described above.

Upon forming flexible circuit 100 into flexible-circuit tip 200 (or 300), a space may be formed between first sector 210 (or 310) and third sector 214 (or 314). This space may be filled with an insulating material, as explained above for the spaces between first sector 110 and second sector 112 and between second sector 112 and third potion 114.

Catheter body 14, outfitted with tip 200 (or 300) thus provides various improvements in catheter tip design. Notably, each sector 210, 212, and 214-and the electronic componentry thereof-is insulated from and functionally independent from the other two sectors. Such assists system 10 to measure and generate information that system 10 or operator 16 may use to provide and modify ablation therapy. In the preferred embodiment, the tip is divided into three or more unique sectors, each having distinct electrodes. The electrodes on one of the sectors can be activated or deactivated separately from the electrodes on each of the other sectors, and they can be activated to provide different functionality, e.g., ablation or ECG sensing. Further, the electrical signals, typically in the RF range of the generator, provided to each of the three sectors may be the same or different than the electrical signals provided to one or both of the others. That is, the power delivered to each tip sector (e.g., power amount denoted in Watts) can be the same or different for each of the sectors. For example, the power amount delivered to the first tip sector ("first power amount" in Watts) can be controlled to be different (i.e., higher or lower) than the power amount delivered to the second tip sector ("second power amount"). As well, the third tip sector can be turned off or a third power amount can be provided to the third tip sector ("third power amount") that is different from the first power amount or the second power amount. Alternatively, energy delivered (in Joules) to each sector can be the same or different for each sector. In yet another example, the frequency of the RF signals provided to one sector may be varied relative to the frequency of the signals provided to one sector or both other sectors. The RF signals may be varied to any frequency within the RF frequency band of 10 kHZ to 1MHz, e.g., based on suitable feedback controls. Such techniques to control energy or power to the tip sectors assist in controlling the temperature of tip 200 or tissue being ablated, and may further assist in improving the precision of the ablation.

It is noted that the make-up of biological tissue (e.g., water content, thickness or other tissue characteristics) in contact with a tip sector can affect the resistivity and therefore the RF power being delivered by that tip sector to the tissue. As such, the amount of temperature rises in that tip sector due to the energy or power delivered to such tissue can be different from other tip sectors in contact with the same tissue at different locations with correspondingly different tissues characteristics (or even different tissues). Therefore, one advantage of the embodiments herein is the ability for the system to deliver different power levels to different tip sectors to ensure that the temperature measured for one tip sector is generally the same for all of the tip sectors.

Tip 200 (or 300) may be brought into contact with tissue such that the tissue contacts at least a portion of the first sector, or at least a portion of the first sector and at least a portion of a second sector, or at least a portion of each of the three sectors. ECG signals may be separately assessed by the various electrodes of the three sectors such that the user or the system can determine which sectors contact tissue to determine which electrodes to activate to ablate. Further, the sector-specific signals of ECG may be used to tailor the therapy. For example, while sector 210, operating as an ablation electrode, provides ablation energy to tissue that sector 210 (or at least a portion thereof) contacts, the temperature sensors on sector 210 measure and provide temperature data to processor 22. Simultaneously, some or all of the temperature sensors on tip 200 may provide temperature data to processor 22, while sectors 212 and 214, operating as electromagnetic sensors and not in contact with tissue, in partial contact with tissue, or in full contact with tissue, may provide ECG data to processor 22 or may be deactivated. Alternatively, one of sectors 212 or 214 may be deactivated while the other provides ECG data. That is, while one or two sectors' electrodes function as ablation electrodes, the other electrodes can provide input to determine if additional areas should be ablated, and if so, how the therapy should be provided or tailored (e.g., via power modification, duration of activation, continuous or pulsed activations, etc.). Further, by providing ablation energy only to those sectors in contact with tissue, ablation energy may be precisely provided directly to tissue such that energy applied to blood may be minimized, which minimizes the likelihood of thrombus formation. In addition, with a smaller area of the anatomy (e.g., epicardial or renal) directly receiving the energy, there will be a higher probability that the errant tissue will be ablated faster and more accurately. Further, the ECG data from non-tissue contacting sector(s) may be used to check for early signs of blockages (e.g., thrombi), while tissue-contacting sector(s) in contact with tissue are being ablated, such that remedial steps may be promptly taken.

Additionally, in certain instances, e.g., when at least a portion of all three sectors is determined to be in contact with tissue, processor 22 may control the application of ablation energy, either automatically or based on user input, such that the ablation energy may be provided to tissue via all three sectors simultaneously or in succession. When the ablation energy is applied in succession to more than one electrode, the ablation electrodes may be activated one at a time or two at a time. Two exemplary in-succession activations include: 1) sector 210 may be activated then deactivate, then sector 212 may be activated then deactivated, and sector 214 may be activated then deactivated; and 2) sectors 210 and 212 may be activated, then sector 210 may be deactivated and sector 214 activated, then sector 212 may be deactivated and sector 210 activated. Additional in-succession activations in differing combinations may be performed and also repeated until the desired ablation is achieved, as indicated by ECG signals or other signals provided by the electrode. One advantage of in-succession activations is that it permits different portions of tissue to be ablated and monitored without moving the catheter. Further, in-succession activations may be combined with simultaneous activations of all of the sectors. Moreover, the activations, whether in sequence or simultaneous, may be performed repeatedly.

In some epicardial applications, certain design considerations may suggest further minimizing heat generated by one sector from being detected by a thermocouple of another sector, and further minimizing the likelihood that ECG signals detected by an electrode on one sector are also detected by a sensor of another sector. Accordingly, one or two of the three sectors may be fabricated with greater insulation properties but without other functions, such as temperature measurement, ablation, and sensing, and associated componentry, such as thermocouples and electrodes. Accordingly, one or two of the sectors, e.g., sector 212, sector 214, or both, may have a greater amount of insulation material incorporated therein than in those embodiments where these sectors include functions of, e.g., ablation. Thus, for example, a ceramic material may be deposited onto flexible circuit 100 over sectors 112 and 114, which assists in preventing heat from ablated tissue from affecting the catheter tip via these sectors.

As noted above, ECG signals may be separately assessed by electrodes disposed on the tip such that the user or the system can determine that the tip contacts tissue, and, in those embodiments with electrodes on different tip sectors, to determine which electrodes to activate for providing ablation therapy. Contact with tissue may also be determined using force contact sensors, e.g., as described in U.S. Patent Application No. 15/452,843, filed March 8, 2017. A contact force sensor particularly suited for use in a catheter having a split tip is now described, and also described in U.S. Patent Application No. 16/036710, filed July 16, 2018.

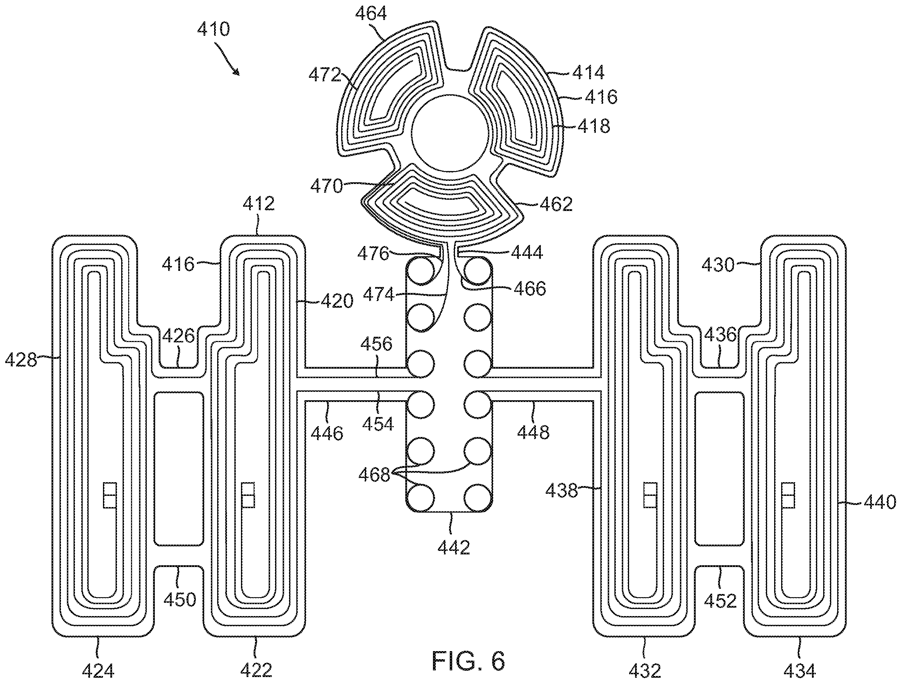

Figure 6 reflects a flexible circuit 410 that may be employed within a catheter, such as catheter 14, to provide signals concerning location and force to a processor in console 24. Flexible circuit 410 includes a substantially planar substrate 412 having a first portion 414 of a first shape (e.g., circular or trefoiled as shown) and a second portion 416 of a second shape (e.g., substantially rectangular or polygonal as shown). First portion 414 and second portion 416 are typically of different shapes because, as will be explained below, second portion 416 is assembled parallel to the longitudinal axis of the catheter, such that it should be elongate, whereas first portion 414 is assembled transversely to the longitudinal axis of the catheter, such that it should conform to the inner diameter of the catheter (i.e., have a maximum width or diameter that is less than or about equal to the inner diameter of the catheter). Nonetheless, shapes of first portion 414 and second portion 416 may be similar. Substrate 412 may be formed of any suitable material that is non-conductive and can resist high temperatures, e.g., polyimide or polyamide.

Substrate 412 may also include additional portions, such as third portion 430 and fourth portion 442. Each of these portions may further include various segments. As noted, first portion 414 may be of a trefoil shape. Thus, it may have three segments, i.e., segments 460, 462, and 464. Second portion 416 may include segment 422 and segment 424, and at least one connector segment, such as 426 or 450, which connect segment 422 to segment 424. Third portion 430 may have a similar structure to second portion 416, and may include segment 432 and segment 434, and at least one connector segment, such as 436 or 452, which connect segment 432 to segment 434. Fourth portion 442 may include at least three connector segments 444, 446 and 448, which connect fourth portion 442 to first, second, and third portions 414, 416, and 430.

Electrical components may be incorporated into substrate 412 and its various portions and segments. For example, substantially planar coils or traces used to measure signals relating to force (i.e., force-sensing coils or traces) may be incorporated onto first portion 414. Specifically, coil 418 may be incorporated with segment 460, coil 470 may be incorporated with segment 462, and coil 472 may be incorporated with segment 464. Coils 418, 470, and 472 may be discrete from each other, as shown, or they may each be connected to one or both of the others. Portions of each coil, or extensions thereof, may extend from the coil to solder joints 468 located on fourth portion 442 and be soldered thereto. Where the three coils are discrete from each other, each should include a respective extension (i.e., 466, 474, and 476). However, where the three coils are connected, only one or two extensions may be necessary. Where the coils are discrete from each other, the signals generated in each of the coils may be used to provide additional details of force, such as an indication of an off-center force or an off-axis direction of the force. Further, catheter 14 may be assembled such that segments 460, 462, and 464 may be aligned, respectively, with sectors 210, 212, and 214. Thus, signals generated in coils 418, 470, and 472 may be used by processor 22 to provide different determinations of force upon each of the sectors. As shown, each coil on first portion 414 includes approximately five turns. However, because signal strength is a function of the number of turns, the number of turns may be maximized based on the size of each segment and the pitch that the lithographic process can accomplish.

Planar coils or traces used to measure signals relating to location (i.e., location coils or traces) may also be incorporated into second portion 416 and third portion 430. Coil 420 may be incorporated with segment 422, coil 428 may be incorporated with segment 424, coil 438 may be incorporated with segment 432, and coil 440 may be incorporated with segment 434. Each of these coils may extend to solder joints 468 on fourth portion 442. For example, coil 420 may include an extension 454 that connects to a solder joint 468 via connector segment 446 and coil 428 may include an extension 456 that connects to a solder joint 468 via connector segment 426, segment 422 and connector segment 446. As shown, each coil on first and second portions 416 and 430 includes approximately five turns. However, because signal strength is a function of the number of turns, the number of turns may be maximized based on the size of segments 422, 424, 432, and 434, and the pitch that the lithographic process can accomplish.

Various symmetries are reflected in Figure 6. For example, the entire substrate is symmetric about a midline passing through the center of first portion 414, such that second portion 416 is laterally disposed to one side of first portion 414 and fourth portion 442 and such that third portion 430 is laterally disposed to the other side of first portion 414 and fourth portion 442. Thus, fourth portion 442 is disposed between first portion 414, second portion 416 and third portion 430. Further, segments 422 and 424 mirror each other, and, with the exception of extension 456, coil 420 mirrors coil 428. The same is true for segments 432 and 434, as well as coils 438 and 440. Accordingly, and as shown, the wind of coils 420 and 432 may be clockwise (i.e., have a clockwise orientation) whereas the wind of coils 428 and 434 may be counterclockwise (i.e., have a counterclockwise orientation). Alternatively, the wind of coils 420 and 432 may be counterclockwise and the wind of coils 428 and 434 may be clockwise.

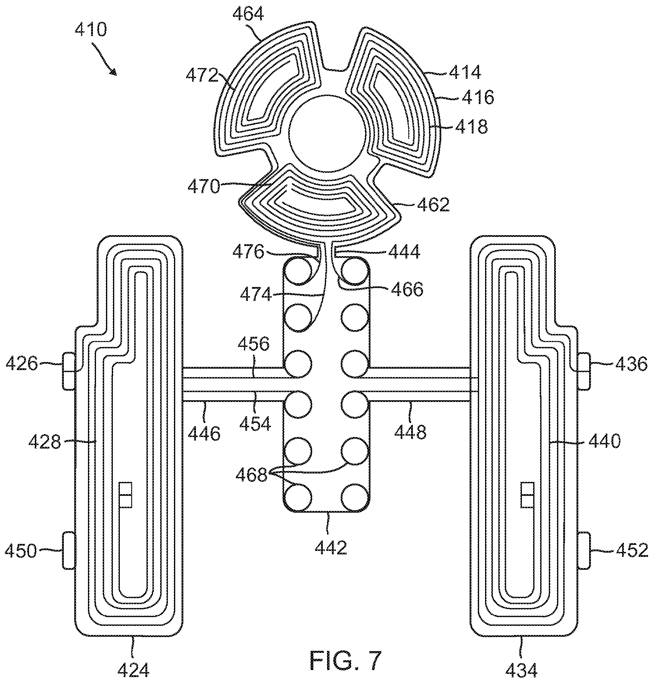

Substrate 412 may be a single layer. Alternatively, it may include between two and ten layers, e.g., four layers. Each layer is identical to the others, including the shapes of the various portions, segments, and coils described above. In this manner the coils may be thickened by adding layers. However, thickening by layers results in increased non-linearity of signal yield. The flexibility of flexible circuit 410 enables a solution to this tradeoff. Specifically, referring to Figure 7, by deforming or bending connector 426 and connector 450, segment 424 may be folded on top of segment 422 to contact it and overlap it such that coil 428 aligns with coil 420. Similarly, by deforming or bending connector 436 and connector 452, segment 434 may be folded on top of segment 432 to contact it and overlap it such that coil 440 aligns with coil 438. Although connectors 450 and 452 are optional, they may assist aligning the coils with each other by reducing relative rotation between the segments. If substrate 412 is four layers, for example, then after segment 424 is folded onto segment 422, coils 420 and 428 form a combined coil having eight layers. The yield of this combined coil does not suffer from increased non-linearity as would the eight-layer coil fabricated in an eight-layer substrate.

An advantage that a thinner substrate (e.g., four layers) has over a thicker substrate (e.g., eight layers) is that it is easier to deform or bend, which is helpful for assembling flexible circuit 410 to other catheter components and ultimately for fitting it within the inner-diameter envelope of the catheter, as will be detailed below. Accordingly, flexible circuit 410 allows for a thick coil without increased non-linearity of the signal and increased stiffness of the substrate.

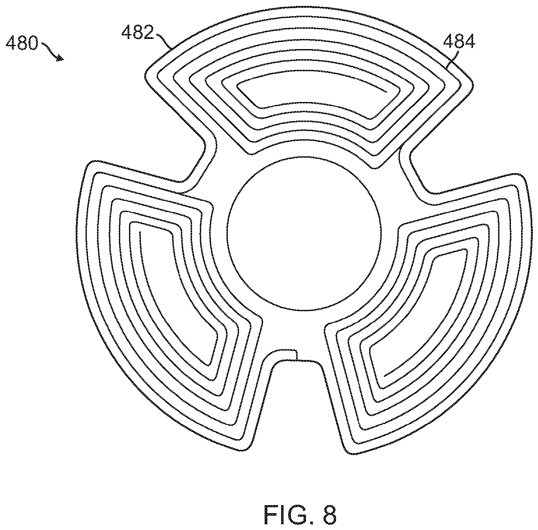

Figure 8 reflects another component of catheter 14, flexible circuit 480, which includes substrate 482 and coil or coils 484. The structure of flexible circuit 480 is similar to the structure of first portion 414 of flexible circuit 410. However, in various embodiments, the number or pitch of the coils may vary, and the various coils on the three segments may be discrete from each other or integrated with each other.

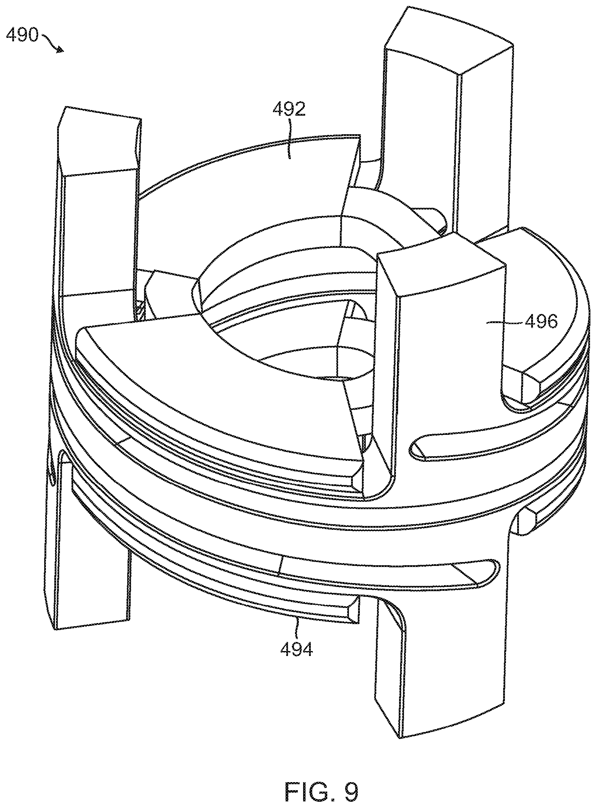

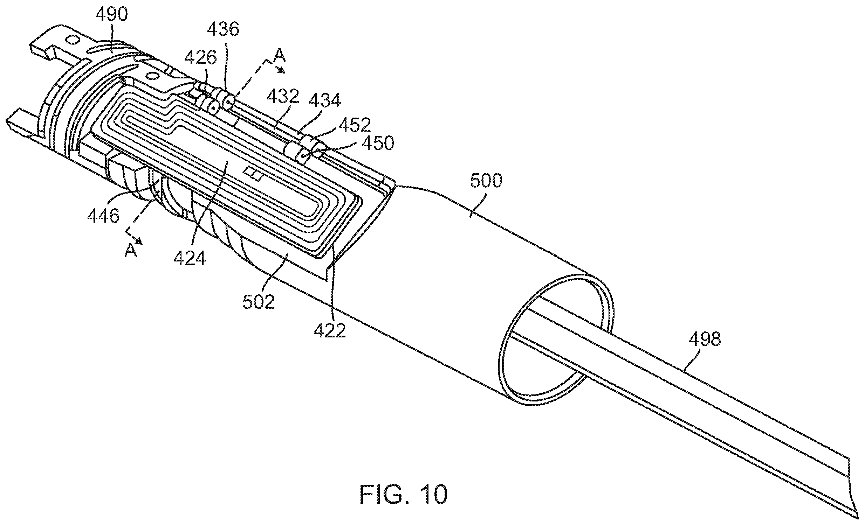

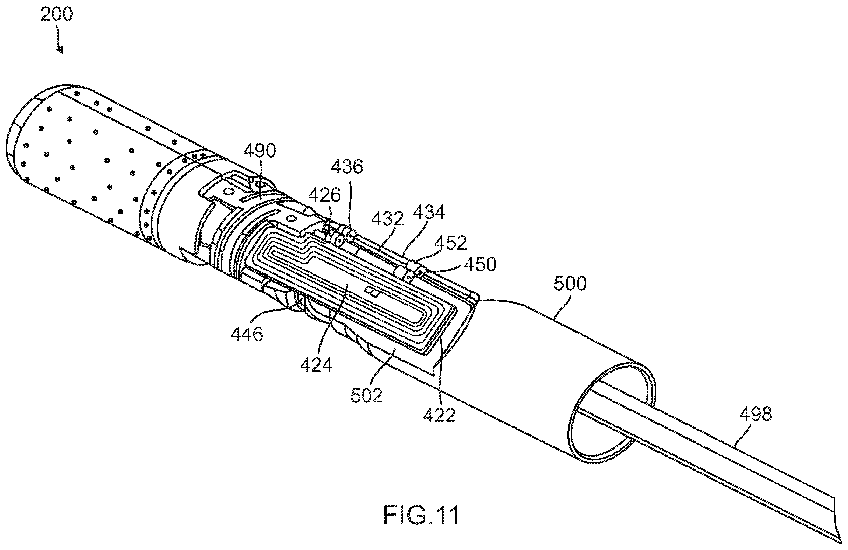

Figure 9 reflects another component of catheter 14, helical spring 490, that includes a top face 492, a bottom face 494, and various arms 496 that may be used to assemble spring 490 to other components of catheter 14. Spring 490 has a known or predetermined spring constant that relates distance to force in accordance with Hooke's law. Together flexible circuit 480, first portion 414 of flexible circuit 410, and helical spring 490 make up a sub-assembly that may receive electrical signals from and provide electrical signals to console 24, which may be processed to determine forces, e.g., sub-gram forces, exerted on tip 18, (or tip 200 or tip 300, as the case may be) of catheter 14. Specifically, a first cable or cables (within cable-bundle 498 of Figures 10 and 11) that connects to console 24 on one end may be also connected at the opposite end to solder joints 468 of fourth portion 442 of flexible circuit 410 that are connected via coil extensions 466, 474, and 476 to coils 418, 470, and 472 on segments 460, 462, and 464 of first portion 414, respectively. A second cable or cables (also within cable-bundle 498) that connects to console 24 on one end may also be connected at the opposite end to coil or coils 484 on flexible circuit 480. Electrical signals from console 24, e.g., having RF frequencies, may be used to power either the coils on the first portion of flexible circuit 410 or the coils on flexible circuit 480. Whichever set of coils receives power from console 24 may be considered a transmitter because it emits an electromagnetic field that varies in accordance with the frequency of the signals received from console 24. The set of coils that is not powered by console 24 may be considered a receiver inasmuch as it functions like an antenna in response to the electromagnetic field from the transmitter. Thus, the receiver generates electrical signals that may be conveyed to console 24 for analysis. The electrical signals generated by the receiver depend on the distance between the receiver and the transmitter, such that the electrical signals generated by the receiver may be correlated to the distance between the receiver and the transmitter.

By adhering the receiver (here, the coils on first portion 414 of flexible circuit 410) to top face 492 of spring 490 and the transmitter (here, the coils on flexible circuit 480) to the bottom face of spring 480, and wiring them as described above, electrical signals generated in the receiver may be correlated to compression displacement in the spring (e.g., on the order of 100 nanometers) and thus to forces against tip 200 or 300 of catheter 14 that cause spring 480 to compress. In use, console 24 with processor 22 may process these signals and use them to confirm that contact has been made between tip and tissue, and to regulate the amount of ablation energy supplied to electrodes. For example, when the signals indicate that the spring is in a relaxed state (i.e., no compression) this may be perceived as an indicator that tip 200 or 300 does not contact tissue, and therefore, no ablation energy should be supplied to the electrodes. Indicators of the information (e.g., in units of force, such as newtons) may further be provided to operator 16 on monitor 29. This information may be useful to provide directly to operator 16 insofar as it may help operator 16 avoid damaging tissue by pressing the tip against it too hard.

Furthermore, each of the three portions of the trefoiled shapes of first portion 414 of flexible circuit 410 and flexible circuit 480 may be aligned with each other and with the three sectors 210, 212, and 214 of tip 200, such that differences in contact force from tissue against the three sectors may be determined by processor 22. Accordingly, processor 22 may determine, e.g., that the greatest contact force against tissue is experienced by sector 210, the second greatest contact force against tissue is experienced by sector 212, and the least contact force against tissue is experienced by sector 214. Thus, processor 212 may use the force data alone or in combination with the ECG data from these sectors, to tailor the RF energy applied to each of the sectors' electrodes for ablating tissue. Further, the user may observe information concerning force of contact of each of the three sectors on display 29 and use it to determine which sectors contact tissue and to adjust the position of tip 200 to achieve desired contact forces against tissue.

Top face 492 and bottom face 494 of spring 490 may be parallel to each other and oriented transversely to the longitudinal axis of the spring (e.g., at an angle of greater than about sixty degrees and less than ninety degrees, e.g., about eighty degrees). Accordingly, the receiver and the transmitter, affixed thereto, are similarly angulated. However, non-perpendicular angulation increases the sensitivity of the receiver because the distance between the transmitter and receiver is minimized as compared to if they were provided perpendicular to the spring's longitudinal axis, and ultimately the catheter's longitudinal axis. Such angulation may further assist in distinguishing relative forces exerted against the three tip sectors.

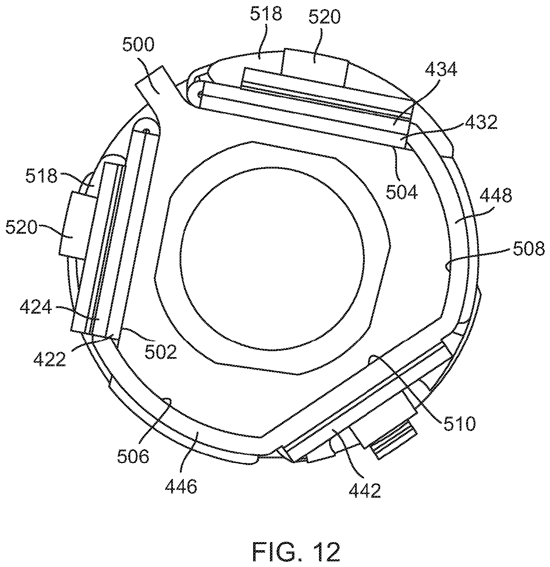

Figures 10 and 11 show catheter 14 at two different steps of its assembly. Figure 12 is a cross section of catheter 14 taken along line A-A in Figure 10, but with various components removed or simplified for clarity concerning further discussion of flexible circuit 410. Figure 10 shows flexible circuit 410 as assembled to spring 490 and a coupling sleeve 500. Although not seen, first portion 414 of flexible circuit 410 is adhered to top face 492 of spring 490 and flexible circuit 480 is adhered to bottom face 494 of spring 490. In Figure 11, tip 200 is shown attached to spring 490. Also shown in Figures 10 and 11 is cable bundle 498. Cable bundle 498 includes a set of cables, which, although not visible, are connected to solder joints 468 on fourth portion 442 of flexible circuit 410, and thus to the various coils or traces on flexible circuit 410, and to coils or traces 484 on flexible circuit 480. As seen in Figures 10-12, flexible circuit 410 is no longer planar. Rather, it has been deformed to have a shape that has a cross section that is partially circular and partially triangular. Segment 424 of second portion 416 is the most readily visible segment of flexible circuit 410 in Figures 10 and 11. Various sides of segment 422, segment 432, and segment 434, as well as connectors 426, 436, 446, 450, and 452 are also visible in these figures. As seen these connectors have been deformed into bent or curved configurations for attachment to coupling sleeve 500. Specifically, segment 422 is adhered to a substantially planar surface 502 of sleeve 500 and segment 432 is adhered to a substantially planar surface 504 of sleeve 500. So assembled, these portions of flexible circuit 410 may be viewed as having a triangular cross section. Further, connector 446 is adhered to a circular (or arcuate) surface 506 of sleeve 500 and connector 448 is adhered to a circular (or arcuate) surface 508 of sleeve 500. So assembled, these portions of flexible circuit 410 may be viewed as having a circular (or arcuate) cross section. Fourth portion 442 may further be adhered to substantially planar surface 510 of sleeve 500.

The diameter or width of the circular portion of the cross section of flexible circuit 410 as assembled to sleeve 500 is equal or approximately equal to the diameter or maximum width of first portion 414, which is also equal or approximately equal to the maximum width (or base) of the triangular portion of the cross-section of flexible circuit 410 as assembled to sleeve 500. Accordingly, as assembled, flexible circuit 410, may be readily inserted into an outer tube or sleeve that provides an outer surface of catheter 14 and that defines the inner diameter within which componentry (e.g., flexible circuit 410, spring 480, sleeve 500) of catheter 14 must fit. To help prevent soft spots under the outer sleeve that result from gaps between the substantially planar outer surfaces of segments 424 and 434, and portion 442 on the one hand, and the curvature of the outer sleeve on the other hand, these gaps may be filled by including additional material, e.g., adhesives 518 and polyimide layers 520, on segments 424 and 434 (of second portion 416 and third portion 430, respectively) and portion 442. The polyimide layers 520 may be fabricated separately from flexible circuit 410 and adhered thereto, or they may be an integral portion of flexible circuit 410, formed during the same lithographic process as the remainder of flexible circuit 410. Polyimide layers 520 may interpolate the curve of the outer sleeve with a series of substantially planar steps or layers.

Flexible circuit 410 may be assembled into catheter 14 as follows. First, flexible circuit 410 may be provided. Segment 424 of second portion 416 may be folded over segment 422 of second portion 416 to overlap it and contact it by deforming connector 426 and, if included, connector 450. Segment 434 of third portion 430 may be folded over segment 432 of third portion 430 to overlap it and contact it by deforming connector 436 and, if included, connector 452. First portion 414 of flexible circuit 410 may be oriented to be parallel to top face 492 of spring 490, which is oriented transversely (e.g., less than thirty degrees from perpendicular) to a longitudinal axis of spring 490. First portion 414 may then be adhered to top face 492 of spring 490. A coupling sleeve 500 having substantially planar surface portions may be provided and oriented to align its longitudinal axis with the longitudinal axis of the spring. Second portion 416 and third portion 430 may be oriented to be parallel to respective substantially planar surface portions of sleeve 500. Then, second portion 416 and third portion 430 may be adhered to the respective substantially planar surface portions of sleeve 500. Sleeve 500, adhered to flexible circuit 410, may then be coupled or inserted into the outer sleeve. Finally, tip 18 may be affixed to spring 490. Flexible circuit 480 may be adhered to bottom face 494 of spring 490 at nearly any step of the process so long as tip 18 has not been attached to spring 490.

By virtue of the embodiments illustrated and described herein, Applicant has devised a method of ablating tissues selectively along a tissue surface, e.g., a curved tissue surface, in contact with a some or all of a flexible-circuit tip of a diagnostic/therapeutic catheter, while using other sectors of the tip, particularly those not in contact with tissue, to provide functions besides ablations, such as monitoring of electromagnetic signals (e.g., ECG signals). That is, a user may use the diagnostic/therapeutic catheter described above or the electrophysiology system of which it may be a part, according to various methods and variations to activate at least one electrode while maintaining inactive the other electrodes or using them to provide functions besides ablations, such as monitoring of electromagnetic signals (e.g., ECG signals), while further measuring temperature using some or all of the various temperature sensors disposed on the catheter tip. One such method and variations may include the following steps. First, a user may receive the catheter. Then the user may introduce the catheter into a subject, e.g., a human subject, and position the catheter proximate to heart tissue. Second, the user may contact the catheter's tip against the heart tissue. Third, a processor connected to the catheter may receive temperature data, ECG signals via the catheter's tip sectors (e.g., 210, 212, 214), and force signals via the catheters receiving and transmission coils (e.g., 118 and 184). The processor may use either the ECG signals, the force signals, or both to determine which the tip sectors are in contact with tissue (at least partially so), and those that are not. Fourth, the processor may then control delivery of ablation energy to only those tips that are in contact with tissue. In those instances where multiple tip sectors contact tissue, the multiple tip sectors in contact with tissue may receive ablation energy, either simultaneously or in succession (as explained above). Similarly, these multiple tip sectors may monitor ECG signals simultaneously or in succession. In an exemplary variation where all three tip sectors contact tissue, tip sector 210 may receive ablation energy while sectors 212 and 214 provide ECG signals to the processor, then sector 210 may be switched from receiving ablation energy to providing ECG signals and sector 212 may be switched from providing ECG signals to receiving ablation energy, then, sector 212 may be switched back to providing ECG signals and sector 214 may be switched from providing ECG signals to receiving ablation energy. In further variations, at least the temperature sensors may provide temperature data to the processor at least while the sector on which they are disposed receives ablation energy. However, all of the temperature sensors may continuously provide temperature data to the processor.

Further, a diagnostic/therapeutic catheter having the features described above may be built in accordance with the following method and variations thereof. First, a flexible circuit (e.g., flexible circuit 100) may be fabricated, e.g., via a lithographic process. For example, a layer of an insulative material (e.g., polyamide) may be deposited, which may be a substrate for electronic componentry. Next, a layer of a conductive material (e.g., platinum or gold) comprising the electronic componentry (e.g., thermocouple 118, conductor element 126, and contact 146) may be deposited. Next, another layer of insulative material may be deposited. Next, another layer of a conductive material may be deposited. Mask layers may also be deposited to achieve particular shapes and configurations of the layers. For example, mask layers may be used to create different segments of the flexible circuit (e.g., segments 102 and 104), sectors thereof (e.g., sectors 110 and 114), and to shape the electronic componentry. A further step of creating ports (e.g., irrigation ports 134) through the flexible circuit (e.g., via laser drilling) may be performed if such ports were not fabricated in the preceding steps.

Second, flexible circuit 100 may be received, along with other components for building the diagnostic/therapeutic catheter, e.g., catheter body 14. Other components may also be received, e.g., a core, such as core 235, and electrodes not fabricated as an integral part of flexible circuit 100, such as electrodes 32. Additionally, flexible circuits 410 and 480 may be received to impart force measurement functionality to the catheter.

Third, flexible circuit 100, which may be received in a planar configuration, may have at least its second segment formed into a non-planar configuration, e.g., a cylindrical configuration. In so doing, the flexible circuit may be formed into a flexible-circuit tip (e.g., 200). The flexible-circuit tip may then be connected, typically by the second segment, to the catheter body. Fourth, in some variations, conductor element(s) (e.g., 132) may be connected to electrodes 32.

Fifth, lead wires may be connected to contacts (e.g., 142) on solder pads (e.g., 136) that are operatively connected to the electronic componentry of the flexible-circuit tip, as well as flexible circuits 410 and 480. Sixth, spaces between the various sectors of the flexible-circuit tip may be filled with an insulation material.

**In** those variations of the method that include providing a core, the flexible circuit may be conformed to the core to change its configuration into that of the flexible-circuit tip. In other variations of the method that include providing a core, the flexible-circuit tip may be attached to the core. Further, an insulation material may be disposed in a space between the core and the flexible-circuit tip.

Any of the examples or embodiments described herein may include various other features in addition to or in lieu of those described above. The teachings, expressions, embodiments, examples, etc., described herein should not be viewed in isolation relative to each other. Various suitable ways in which the teachings herein may be combined should be clear to those skilled in the art in view of the teachings herein.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but in any order as long as the steps allow the embodiments to function for their intended purposes. Therefore, to the extent there are variations of the invention, which are equivalent to the inventions found in the claims, it is the intent that this patent will
cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. A flexible circuit (100) configured to be formed into a cylindrical configuration, comprising:
a first planar segment (102) and a second planar segment (104),
wherein the first planar segment is configured to be formed into a base of the cylindrical configuration and the second planar segment is configured to be formed into a wall of the cylindrical configuration;
wherein the second planar segment (104) comprising a plurality of irrigation ports (134) disposed therethrough,
the second planar segment (104) further comprising
a first layer including a substrate,
a second layer including at least a first temperature sensor (116), a second temperature sensor (118), a third temperature sensor (122), a fourth temperature sensor (124), and a conductor element (120, 126, 132),
a third layer including an insulator,
a first sector (110) having the first temperature sensor (116) and the second temperature sensor (118),
a second sector (112) having the third temperature sensor (122) and the fourth temperature sensor (124), and
a first space provided between the first sector (110) and the second sector (112) through the second layer.

2. The flexible circuit (100) of claim 1, wherein the second planar segment (104) further includes a third sector (114), the third sector (114) having a fifth temperature sensor (128) and a sixth temperature sensor (130).

3. The flexible circuit (100) of any preceding claim, in which the conductor element includes a trace (120, 126, 132) connected to an ablation electrode.

4. The flexible circuit (100) of claim 3, when dependent on claim 2, in which the first sector (110), the second sector (112), and the third sector (114) each include a respective solder pad (136, 138, 140) having a first contact (142) operatively coupled to a respective thermocouple, a second contact (144) operatively coupled to another respective thermocouple, and a third contact (146) operatively coupled to a respective electrode.

5. The flexible circuit (100) of any preceding claim, in which the first planar segment (102) includes a first-segment substrate and a first-segment insulator, optionally wherein the first planar segment (102) further includes a first-segment temperature sensor, and further optionally wherein the first planar segment (102) further includes a first-segment electrode.

6. The flexible circuit of claim 1, in which the insulator comprises polyamide, polyimide, liquid crystal polymer, or polyurethane.

7. The flexible circuit (100) of claim 2, further comprising a second space between the second layer of the second sector (112) and the second layer of the third sector (114), optionally wherein the flexible circuit comprises a first insulation material disposed within the first space and the second space, and further optionally wherein the first insulation material includes a high-temperature epoxy.

8. The flexible circuit (100) of claim 1, the first sector (110) additionally having an ablation electrode, and the second sector (112) covered in an insulation material, optionally wherein the second planar segment (104) includes a third sector (114) covered in an insulation material, and further optionally wherein the insulation material is ceramic.

9. A catheter, comprising:
an elongate catheter body (14) having at least two lumens disposed longitudinally therethrough and a distal end (15) ; and
a flexible-circuit tip (200) connected to the distal end, the flexible circuit tip (200) comprising the flexible circuit (100) of any one of claims 1 to 8.

10. The catheter of claim 9, further comprising a core (250) attached to the distal end (15) of the catheter, at least a portion of which is disposed within the second segment (104) of the flexible-circuit tip (200), optionally wherein the core (250) comprises an insulative material, and further optionally wherein the insulative material comprises polycarbonate.

11. The catheter of claim 10, in which the core (250) includes a lumen oriented transverse to a longitudinal axis of the core (250).

12. The catheter of claim 10, further comprising a second insulation material disposed between the second segment (104) and the core (250).

13. The catheter of claim 10, in which the core (250) is in communication with a first one of the at least two lumens of the catheter body, optionally wherein the catheter further comprises a plurality of wires disposed within at least a second one of the at least two lumens, the plurality of wires electrically connected to the flexible-circuit tip (200).

14. A method of assembling a catheter, comprising:
receiving a catheter body including a distal end,
receiving a flexible-circuit tip (200) including
a first planar segment (102) and a second planar segment (104),
wherein the first planar segment is configured to be formed into a base of the cylindrical configuration and the second planar segment is configured to be formed into a wall of the cylindrical configuration and the second planar segment (104) comprising a plurality of irrigation ports (134) disposed therethrough,
the second planar segment (104) further comprising
a first layer including a substrate,
a second layer including at least a first temperature sensor (116), a second temperature sensor (118), a third temperature sensor (122), a fourth temperature sensor (124), and a conductor element (120, 126, 132),
a third layer including an insulator;
a first sector (110), and a second sector (112), the first sector (110) having the first temperature sensor and the second temperature sensor, and the second sector (112) having the third temperature sensor and the fourth temperature sensor; and
a first space provided between the first sector (110) and the second sector (112) through the second layer;
changing the planar configuration of the second planar segment (104) to a non-planar configuration, wherein the non-planar configuration includes a cylindrical configuration; and
connecting the second planar segment (104) in the non-planar configuration to the distal end of the catheter body.

15. The method of claim 14, in which the conductor element includes a trace connected to an ablation electrode, and wherein the method comprises connecting the conductor element (120, 126, 132) to an electrode.

16. The method of claim 15, wherein the second planar segment (104) further includes a third sector (114), the third sector (114) having a fifth temperature sensor (128) and a sixth temperature sensor (130), in which the first sector (110), the second sector (112), and the third sector (114) each include a respective solder pad (136, 138, 140) having a first contact (142) operatively coupled to a respective thermocouple, a second contact (144) operatively coupled to another respective thermocouple, and a third contact (146) operatively coupled to a respective electrode, and wherein the method further comprises connecting lead wires to the first contact (142), the second contact (144), and the third contact (144) of each of the first sector (110), the second sector (112), and the third sector (114).

17. The method of claim 14, wherein the second planar segment (104) further includes a third sector (114), the third sector (114) having a fifth temperature sensor (128) and a sixth temperature sensor (130), in which the second segment (104) further comprises a second space between the second sector (112) and the third sector (114), and a third space between the third sector (114) and the first sector (110), and in which the second segment (104) in the non-planar configuration comprises a third space between the third sector (114) and the first sector (110), and wherein the method further comprises disposing a first insulation material in the first space, the second space, and the third space.

18. The method of claim 17, further comprising:
receiving a core (250);
attaching the core (250) to the distal end of the catheter body;
disposing the core (250) within a portion of the second segment (104) of the flexible-circuit tip (200) in the cylindrical configuration; and
attaching the flexible-circuit tip (200) to the core (250), and optionally disposing a second insulation material between the second segment (104) and the core (250).

## Patentansprüche

1. Flexible Schaltung (100), die konfiguriert ist, um zu einer zylindrischen Konfiguration ausgebildet zu werden, umfassend:
ein erstes planares Segment (102) und ein zweites planares Segment (104),
wobei das erste planare Segment konfiguriert ist, um zu einer Basis der zylindrischen Konfiguration ausgebildet zu werden, und das zweite planare Segment konfiguriert ist, um zu einer Wand der zylindrischen Konfiguration ausgebildet zu werden;
das zweite planare Segment (104) umfassend eine Vielzahl von dahindurch angeordneten Spülöffnungen (134),
das zweite planare Segment (104) ferner umfassend
eine erste Schicht, die ein Substrat einschließt,
eine zweite Schicht, die mindestens einen ersten Temperaturfühler (116), einen zweiten Temperaturfühler (118), einen dritten Temperaturfühler (122), einen vierten Temperaturfühler (124) und ein Leiterelement (120, 126, 132) einschließt,
eine dritte Schicht, die einen Isolator einschließt,
einen ersten Sektor (110), der den ersten Temperaturfühler (116) und den zweiten Temperaturfühler (118) aufweist,
einen zweiten Sektor (112), der den dritten Temperaturfühler (122) und den vierten Temperaturfühler (124) aufweist, und
einen ersten Raum, bereitgestellt zwischen dem ersten Sektor (110) und dem zweiten Sektor (112) durch die zweite Schicht.

2. Flexible Schaltung (100) nach Anspruch 1, wobei das zweite planare Segment (104) ferner einen dritten Sektor (114) einschließt, wobei der dritte Sektor (114) einen fünften Temperaturfühler (128) und einen sechsten Temperaturfühler (130) aufweist.

3. Flexible Schaltung (100) nach einem der vorstehenden Ansprüche, wobei das Leiterelement eine Bahn (120, 126, 132) einschließt, die mit einer Ablationselektrode verbunden ist.

4. Flexible Schaltung (100) nach Anspruch 3, wenn abhängig von Anspruch 2, wobei der erste Sektor (110), der zweite Sektor (112) und der dritte Sektor (114) jeweils ein entsprechendes Lötpad (136, 138, 140) einschließen, das einen ersten Kontakt (142), der mit einem entsprechenden Thermoelement wirkgekoppelt ist, einen zweiten Kontakt (144), der mit einem anderen entsprechenden Thermoelement wirkgekoppelt ist, und einen dritten Kontakt (146), der mit einer entsprechenden Elektrode wirkgekoppelt ist, aufweist.

5. Flexible Schaltung (100) nach einem der vorstehenden Ansprüche, wobei das erste planare Segment (102) ein Erstsegmentsubstrat und einen Erstsegmentisolator einschließt, optional wobei das erste planare Segment (102) ferner einen Erstsegmenttemperaturfühler einschließt, und ferner optional wobei das erste planare Segment (102) ferner eine Erstsegmentelektrode einschließt.

6. Flexible Schaltung nach Anspruch 1, wobei der Isolator Polyamid, Polyimid, Flüssigkristallpolymer oder Polyurethan umfasst.

7. Flexible Schaltung (100) nach Anspruch 2, ferner umfassend einen zweiten Raum zwischen der zweiten Schicht des zweiten Sektors (112) und der zweiten Schicht des dritten Sektors (114), optional wobei die flexible Schaltung ein erstes Isoliermaterial umfasst, das innerhalb des ersten Raums und des zweiten Raums angeordnet ist, und ferner optional wobei das erste Isoliermaterial ein Hochtemperaturepoxidharz einschließt.

8. Flexible Schaltung (100) nach Anspruch 1, wobei der erste Sektor (110) zusätzlich eine Ablationselektrode aufweist und der zweite Sektor (112) mit einem Isoliermaterial bedeckt ist, optional wobei das zweite planare Segment (104) einen dritten Sektor (114) einschließt, der mit einem Isoliermaterial bedeckt ist, und ferner optional wobei das Isoliermaterial Keramik ist.

9. Katheter, umfassend:
einen länglichen Katheterkörper (14), der mindestens zwei Lumina, die in Längsrichtung dahindurch angeordnet sind, und ein distales Ende (15) aufweist; und
eine Spitze (200) der flexiblen Schaltung, die mit dem distalen Ende verbunden ist, die Spitze (200) der flexiblen Schaltung umfassend die flexible Schaltung (100) nach einem der Ansprüche 1 bis 8.

10. Katheter nach Anspruch 9, ferner umfassend einen Kern (250), der an dem distalen Ende (15) des Katheters befestigt ist, von dem mindestens ein Abschnitt innerhalb des zweiten Segments (104) der Spitze (200) der flexiblen Schaltung angeordnet ist, optional wobei der Kern (250) ein Isoliermaterial umfasst, und ferner optional wobei das Isoliermaterial Polycarbonat umfasst.

11. Katheter nach Anspruch 10, wobei der Kern (250) ein Lumen einschließt, das quer zu einer Längsachse des Kerns (250) ausgerichtet ist.

12. Katheter nach Anspruch 10, ferner umfassend ein zweites Isoliermaterial, das zwischen dem zweiten Segment (104) und dem Kern (250) angeordnet ist.

13. Katheter nach Anspruch 10, wobei der Kern (250) in Verbindung mit einem ersten der mindestens zwei Lumina des Katheterkörpers steht, optional wobei der Katheter ferner eine Vielzahl von Drähten umfasst, die innerhalb mindestens eines zweiten der mindestens zwei Lumina angeordnet ist, wobei die Vielzahl von Drähten mit der Spitze (200) der flexiblen Schaltung elektrisch verbunden ist.

14. Verfahren zum Zusammenfügen eines Katheters, umfassend:
Aufnehmen eines Katheterkörpers, der ein distales Ende einschließt,
Aufnehmen einer Spitze (200) der flexiblen Schaltung, die einschließt
ein erstes planares Segment (102) und ein zweites planares Segment (104),
wobei das erste planare Segment konfiguriert ist, um zu einer Basis der zylindrischen Konfiguration ausgebildet zu werden, und das zweite planare Segment konfiguriert ist, um zu einer Wand der zylindrischen Konfiguration ausgebildet zu werden, und das zweite planare Segment (104) umfassend eine Vielzahl von dahindurch angeordneten Spülöffnungen (134),
das zweite planare Segment (104) ferner umfassend
eine erste Schicht, die ein Substrat einschließt,
eine zweite Schicht, die mindestens einen ersten Temperaturfühler (116), einen zweiten Temperaturfühler (118), einen dritten Temperaturfühler (122), einen vierten Temperaturfühler (124) und ein Leiterelement (120, 126, 132) einschließt,
eine dritte Schicht, die einen Isolator einschließt;
einen ersten Sektor (110) und einen zweiten Sektor (112), wobei der erste Sektor (110) den ersten Temperaturfühler und den zweiten Temperaturfühler aufweist und der zweite Sektor (112) den dritten Temperaturfühler und den vierten Temperaturfühler aufweist; und
einen ersten Raum, der zwischen dem ersten Sektor (110) und dem zweiten Sektor (112) durch die zweite Schicht bereitgestellt ist;
Ändern der planaren Konfiguration des zweiten planaren Segments (104) in eine nicht planare Konfiguration, wobei die nicht planare Konfiguration eine zylindrische Konfiguration einschließt; und
Verbinden des zweiten planaren Segments (104) in der nicht planaren Konfiguration mit dem distalen Ende des Katheterkörpers.

15. Verfahren nach Anspruch 14, wobei das Leiterelement eine Bahn einschließt, die mit einer Ablationselektrode verbunden ist, und wobei das Verfahren das Verbinden des Leiterelements (120, 126, 132) mit einer Elektrode umfasst.

16. Verfahren nach Anspruch 15, wobei das zweite planare Segment (104) ferner einen dritten Sektor (114) einschließt, wobei der dritte Sektor (114) einen fünften Temperaturfühler (128) und einen sechsten Temperaturfühler (130) aufweist, wobei der erste Sektor (110), der zweite Sektor (112) und der dritte Sektor (114) jeweils ein entsprechendes Lötpad (136, 138, 140) einschließen, das einen ersten Kontakt (142), der mit einem entsprechenden Thermoelement wirkgekoppelt ist, einen zweiten Kontakt (144), der mit einem anderen entsprechenden Thermoelement wirkgekoppelt ist, und einen dritten Kontakt (146), der mit einer entsprechenden Elektrode wirkgekoppelt ist, aufweist, und wobei das Verfahren ferner das Verbinden von Anschlussdrähten mit dem ersten Kontakt (142), dem zweiten Kontakt (144) und dem dritten Kontakt (144) jedes des ersten Sektors (110), des zweiten Sektors (112) und des dritten Sektors (114) umfasst.

17. Verfahren nach Anspruch 14, wobei das zweite planare Segment (104) ferner einen dritten Sektor (114) einschließt, wobei der dritte Sektor (114) einen fünften Temperaturfühler (128) und einen sechsten Temperaturfühler (130) aufweist, wobei das zweite Segment (104) ferner einen zweiten Raum zwischen dem zweiten Sektor (112) und dem dritten Sektor (114) und einen dritten Raum zwischen dem dritten Sektor (114) und dem ersten Sektor (110) umfasst, und wobei das zweite Segment (104) in der nicht planaren Konfiguration einen dritten Raum zwischen dem dritten Sektor (114) und dem ersten Sektor (110) umfasst, und wobei das Verfahren ferner das Anordnen eines ersten Isoliermaterials in dem ersten Raum, dem zweiten Raum und dem dritten Raum umfasst.

18. Verfahren nach Anspruch 17, ferner umfassend:
Aufnehmen eines Kerns (250);
Befestigen des Kerns (250) an dem distalen Ende des Katheterkörpers;
Anordnen des Kerns (250) innerhalb eines Abschnitts des zweiten Segments (104) der Spitze (200) der flexiblen Schaltung in der zylindrischen Konfiguration; und
Befestigen der Spitze (200) der flexiblen Schaltung an dem Kern (250) und optionales Anordnen eines zweiten Isoliermaterials zwischen dem zweiten Segment (104) und dem Kern (250).

## Revendications

1. Circuit flexible (100) conçu pour être formé en une configuration cylindrique, comprenant :
un premier segment plan (102) et un second segment plan (104),
dans lequel le premier segment plan est conçu pour être formé en une base de la configuration cylindrique et le second segment plan est conçu pour être formé en une paroi de la configuration cylindrique ;
dans lequel le second segment plan (104) comprend une pluralité d'orifices d'irrigation (134) disposés à travers celui-ci,
le second segment plan (104) comprenant en outre
une première couche comportant un substrat,
une deuxième couche comportant au moins un premier capteur de température (116), un deuxième capteur de température (118), un troisième capteur de température (122), un quatrième capteur de température (124) et un élément conducteur (120, 126, 132),
une troisième couche comportant un isolant,
un premier secteur (110) ayant le premier capteur de température (116) et le deuxième capteur de température (118),
un deuxième secteur (112) ayant le troisième capteur de température (122) et le quatrième capteur de température (124), et
un premier espace fourni entre le premier secteur (110) et le deuxième secteur (112) à travers la deuxième couche.

2. Circuit flexible (100) selon la revendication 1, dans lequel le second segment plan (104) comporte en outre un troisième secteur (114), le troisième secteur (114) ayant un cinquième capteur de température (128) et un sixième capteur de température (130).

3. Circuit flexible (100) selon l'une quelconque revendication précédente, dans lequel l'élément conducteur comporte un tracé (120, 126, 132) connecté à une électrode d'ablation.

4. Circuit flexible (100) selon la revendication 3, prise en dépendance de la revendication 2, dans lequel le premier secteur (110), le deuxième secteur (112) et le troisième secteur (114) comportent chacun un plot de soudure (136, 138, 140) respectif ayant un premier contact (142) couplé fonctionnellement à un thermocouple respectif, un deuxième contact (144) couplé fonctionnellement à un autre thermocouple respectif, et un troisième contact (146) couplé fonctionnellement à une électrode respective.

5. Circuit flexible (100) selon l'une quelconque revendication précédente, dans lequel le premier segment plan (102) comporte un substrat de premier segment et un isolant de premier segment, facultativement dans lequel le premier segment plan (102) comporte en outre un capteur de température de premier segment, et facultativement en outre dans lequel le premier segment plan (102) comporte en outre une électrode de premier segment.

6. Circuit flexible selon la revendication 1, dans lequel l'isolant comprend du polyamide, du polyimide, un polymère à cristaux liquides, ou du polyuréthane.

7. Circuit flexible (100) selon la revendication 2, comprenant en outre un second espace entre la deuxième couche du deuxième secteur (112) et la deuxième couche du troisième secteur (114), facultativement dans lequel le circuit flexible comprend un premier matériau isolant disposé au sein du premier espace et du second espace, et facultativement en outre dans lequel le premier matériau isolant comporte un époxy haute température.

8. Circuit flexible (100) selon la revendication 1, le premier secteur (110) ayant en complément une électrode d'ablation, et le deuxième secteur (112) couvert dans un matériau isolant, facultativement dans lequel le second segment plan (104) comporte un troisième secteur (114) couvert dans un matériau isolant, et facultativement en outre dans lequel le matériau isolant est de la céramique.

9. Cathéter, comprenant :
un corps de cathéter allongé (14) ayant au moins deux lumières disposées longitudinalement à travers celui-ci, et une extrémité distale (15) ; et
une pointe à circuit flexible (200) reliée à l'extrémité distale, la pointe à circuit flexible (200) comprenant le circuit flexible (100) selon l'une quelconque des revendications 1 à 8.

10. Cathéter selon la revendication 9, comprenant en outre un cœur (250) fixé à l'extrémité distale (15) du cathéter, dont au moins une partie est disposée au sein du second segment (104) de la pointe à circuit flexible (200), facultativement dans lequel le cœur (250) comprend un matériau isolant, et facultativement en outre dans lequel le matériau isolant comprend du polycarbonate.

11. Cathéter selon la revendication 10, dans lequel le cœur (250) comporte une lumière orientée transversale à un axe longitudinal du cœur (250).

12. Cathéter selon la revendication 10, comprenant en outre un second matériau isolant disposé entre le second segment (104) et le cœur (250).

13. Cathéter selon la revendication 10, dans lequel le cœur (250) est en communication avec une première des au moins deux lumières du corps de cathéter, facultativement dans lequel le cathéter comprend en outre une pluralité de fils disposés au sein d'au moins une seconde des au moins deux lumières, la pluralité de fils étant connectés électriquement à la pointe à circuit flexible (200).

14. Procédé d'assemblage d'un cathéter, comprenant :
la réception d'un corps de cathéter comportant une extrémité distale,
la réception d'une pointe à circuit flexible (200) comportant
un premier segment plan (102) et un second segment plan (104),
dans lequel le premier segment plan est conçu pour être formé en une base de la configuration cylindrique et le second segment plan est conçu pour être formé en une paroi de la configuration cylindrique, et le second segment plan (104) comprend une pluralité d'orifices d'irrigation (134) disposés à travers celui-ci,
le second segment plan (104) comprenant en outre
une première couche comportant un substrat,
une deuxième couche comportant au moins un premier capteur de température (116), un deuxième capteur de température (118), un troisième capteur de température (122), un quatrième capteur de température (124) et un élément conducteur (120, 126, 132),
une troisième couche comportant un isolant ;
un premier secteur (110), et un deuxième secteur (112), le premier secteur (110) ayant le premier capteur de température et le deuxième capteur de température, et le deuxième secteur (112) ayant le troisième capteur de température et le quatrième capteur de température ; et
un premier espace fourni entre le premier secteur (110) et le deuxième secteur (112) à travers la deuxième couche ;
le changement de la configuration plane du second segment plan (104) vers une configuration non plane, dans lequel la configuration non plane comporte une configuration cylindrique ; et
la liaison du second segment plan (104) dans la configuration non plane à l'extrémité distale du corps de cathéter.

15. Procédé selon la revendication 14, dans lequel l'élément conducteur comporte un tracé connecté à une électrode d'ablation, et dans lequel le procédé comprend la connexion de l'élément conducteur (120, 126, 132) à une électrode.

16. Procédé selon la revendication 15, dans lequel le second segment plan (104) comporte en outre un troisième secteur (114), le troisième secteur (114) ayant un cinquième capteur de température (128) et un sixième capteur de température (130), dans lequel le premier secteur (110), le deuxième secteur (112) et le troisième secteur (114) comportent chacun un plot de soudure (136, 138, 140) respectif ayant un premier contact (142) couplé fonctionnellement à un thermocouple respectif, un deuxième contact (144) couplé fonctionnellement à un autre thermocouple respectif, et un troisième contact (146) couplé fonctionnellement à une électrode respective, et dans lequel le procédé comprend en outre la connexion de fils conducteurs au premier contact (142), au deuxième contact (144) et au troisième contact (144) de chacun du premier secteur (110), du deuxième secteur (112) et du troisième secteur (114).

17. Procédé selon la revendication 14, dans lequel le second segment plan (104) comporte en outre un troisième secteur (114), le troisième secteur (114) ayant un cinquième capteur de température (128) et un sixième capteur de température (130), dans lequel le second segment (104) comprend en outre un second espace entre le deuxième secteur (112) et le troisième secteur (114), et un troisième espace entre le troisième secteur (114) et le premier secteur (110), et dans lequel le second segment (104) dans la configuration non plane comprend un troisième espace entre le troisième secteur (114) et le premier secteur (110), et dans lequel le procédé comprend en outre le fait de disposer un premier matériau isolant dans le premier espace, le second espace et le troisième espace.

18. Procédé selon la revendication 17, comprenant en outre :
la réception d'un cœur (250) ;
la fixation du cœur (250) à l'extrémité distale du corps de cathéter ;
le fait de disposer le cœur (250) au sein d'une partie du second segment (104) de la pointe à circuit flexible (200) dans la configuration cylindrique ; et
la fixation de la pointe à circuit flexible (200) au cœur (250), et le fait disposer facultativement un second matériau isolant entre le second segment (104) et le cœur (250).
